Europäisches Patentamt

European Patent Office

Office européen des brevets

(11) Veröffentlichungsnummer: **0 157 122**
**B1**

(12) **EUROPÄISCHE PATENTSCHRIFT**

(45) Veröffentlichungstag der Patentschrift:
**14.01.87**

(21) Anmeldenummer: **85101271.6**

(22) Anmeldetag: **07.02.85**

(51) Int. Cl.⁴: **C 07 C 67/08,** C 07 C 69/82,
C 07 C 51/09, C 07 C 63/26

(54) **Verfahren zur Herstellung von Terephthalsäure über Terephthalsäuredimethylester aus p-Xylol und Methanol.**

(30) Priorität: **03.03.84 DE 3407912**

(43) Veröffentlichungstag der Anmeldung:
**09.10.85 Patentblatt 85/41**

(45) Bekanntmachung des Hinweises auf die Patenterteilung:
**14.01.87 Patentblatt 87/3**

(84) Benannte Vertragsstaaten:
**BE DE FR GB IT NL**

(56) Entgegenhaltungen:
**GB-A-878 063**

(73) Patentinhaber: **DYNAMIT NOBEL
AKTIENGESELLSCHAFT, Postfach 1261, D-5210
Troisdorf, Bez. Köln (DE)**

(72) Erfinder: **Modic, Rudolf, Dr., Waldfrieden 8, D-3074
Steyerberg (DE)**
Erfinder: **Porschen, Jörg, Merzenicher Strasse 154,
D-5160 Düren (DE)**
Erfinder: **Schoengen, Anton, In den Espeln 9,
D-5810 Witten (DE)**
Erfinder: **Wirges, Ralf, Porzer Strasse 36, D-5216
Niederkassel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Terephthalsäure über Terephthalsäuredimethyleater aus p-Xylol und Methanol der in Oberbegriff von Patentanspruch 1 bezeichneten Art.

Bei diesem Verfahren (vgl. Hydrocarbon Processing, Nov. 1983, Seiten 91 und 151) wird in einem Veresterungsreaktor das aus der Oxidation kommende Oxidat mit Methanol zu Rohester verestert. Das Gewichtsverhältnis von Methanol zu Oxidat beträgt dabei etwa zwischen 0,2 : 1 und 1,0 : 1. Von der bei großtechnischer Durchführung in den Veresterungsreaktor gefahrenen Methanolmenge werden nur etwa 30 bis 50 % für die Veresterungsreaktion benötigt. Das restliche Methanol dient dazu, das Veresterungsgleich gewicht in Richtung einer möglichst vollständigen Veresterung des Oxidats zu verschieben, des weiteren als Energie träger und als Trägermedium für das bei der Veresterungsreaktion entstehende Wasser. Der in die Veresterung eingesetzte Methanoldampf wird um etwa 0 bis 50°C über Veresterungstemperatur überhitzt in die Veresterung eingespeist.

Die Hydrolysereaktion wird z.B. in einem mehrstufigen Reaktor so durchgeführt, daß 350 bis 140°C heißes Roh-DMT am Kopf und 180 bis 350°C heißer Wasserdampf am Boden des Hydrolysereaktors eingeleitet und bei einem Umsatz von über 90 % am Boden eine terephthalsäurehaltige wäßrige Phase und am Kopf des Reaktors ein Methanol-Wasser-Gemisch abgezogen werden (vgl. DE-OS 30 44 617).

Bei der bisher ausgeübten Arbeitsweise wurde aus der in der Hydrolysereaktion anfallenden methanol-haltigen wäßrigen Mutterlauge durch Destillation ein Methanol-Wasser-Gemisch zurückgewonnen. Die bei der Rektifikation des Gemisches entstehenden Brüden wurden kondensiert und in den Vorratstank für Reaktionsmethanol gepumpt. Das Veresterungsmethanol wurde dem Behälter für Reaktionsmethanol entnommen, über Hochdruckpumpen auf beispielsweise einen Druck von 27 bar gebracht, bei diesem Druck aufgeheizt und verdampft, anschließend auf beispielsweise etwa 250 bis 270°C überhitzt und der Veresterung zugeführt.

Der Nachteil dieser Verfahrensweise ist, daß das Veresterungsmethanol somit zweimal verdampft wird.

Es bestand ein erhebliches wirtschaftliches Interesse an einer Verbesserung der Reaktionsmethanolversorgung, welche zu einem energiemäßig günstigeren Verfahren zur Herstellung von Terephthalsäure in großtechnischen Anlagen führt als das mit einer zweimaligen Methanolverdampfung arbeitende bekannte System.

Gemäß der Erfindung wird diese Aufgabe bei dem Verfahren der eingangs angegebenen Art dadurch gelöst, daß die Merkmale des kennzeichnenden Teils von Patentanspruch 1 verwirklicht sind.

Das vorgeschlagene System der Versorgung der Veresterung mit Reaktionsmethanol auf dem benötigten Druck- und Temperaturniveau wird so durchgeführt, daß das Beaktionsmethanol durch Rektifikation der methanol-haltigen Brüdenfraktion aus der Veresterung und anderer methanol-haltiger Prozeßströme sowie des methanol-haltigen Prozeßstromes aus der Hydrolyse unter einsm Druck, der unterhalb des Veresterungsdruckes liegt, gewonnen wird und daß die Methanolbrüden anschließend auf den bei der Veresterung angewandten Druck komprimiert werden, wobei die Kompressionswärme zur Temperaturerhöhung des Methanoldampfes auf die bei der Veresterung benötigte Temperatur ausgenutzt wird. Der zur Veresterung eingesetzte methanolhaltige Dampf wird aus eingesetzten Frisch-Methanol, das zum Ausgleich der Methanolverluste dient, durch Verdampfung sowie durch Rektifikation der methanol-haltigen Brüdenfraktion aus der Veresterung und anderer methanolhaltiger Prozeßströme sowie des methanol-haltigen Prozeßstromes aus der Hydrolyse bei einem Druck von 2 bis 20 bar, vorzugsweise 4 bis 8 bar, erzeugt. Das eingesetzte Frisch-Methanol wird dabei zweckmäßig unter dem gleichen Druck wie er bei der Rektifikation der methanolhaltigen Brüdenfraktion aus der Veresterung und anderer methanol-haltiger Prozeßströme sowie des methanol-haltige Prozeßstromes aus der Hydrolyse herrscht, verdampft. Das einzusetzende Frisch-Methanol kann aber auch direkt in die Rektifikation der methanol-haltigen Brüdenfraktion aus der Veresterung und anderer methanol-haltiger Prozeßströme sowie des methanol-haltigen Prozeßstromes aus der Hydrolyse eingespeist werden. Das Verdichtungsverhältnis des der Kompression unterworfenen methanol-haltigen Dampfes wird im allgemeinen auf einen Wert zwischen 1,2 : 1 und 15 : 1, vorzugsweise 3 : 1 und 9 : 1, eingestellt, wobei die bei der Kompression erreichten Endtemperaturen des den Verdichter verlassenden Reaktionsmethanols bei einer Temperatur zwischen 150 und 300, vorzugsweise 220 bis 280°C, liegen.

Die Energie der aus der Veresterung am Kopf des Veresterungsreaktors austretenden Brüden aus Überschußmethanol und Reaktionswasser kann vorteilhaft nach einer Waschung mit Prozeßwasser oder auch dephlegmiertem Rücklauf des Veresterungsreaktors durch Entspannung in einer Turbine genutzt werden.

Der Haupteffekt bei dem vorgeschlagenen neuen Verfahren besteht darin, daß wertvolle Primärenergie in Form von hochgespanntem Dampf (17 bis 25 bar) oder Wärmeträgern eingespart werden kann.

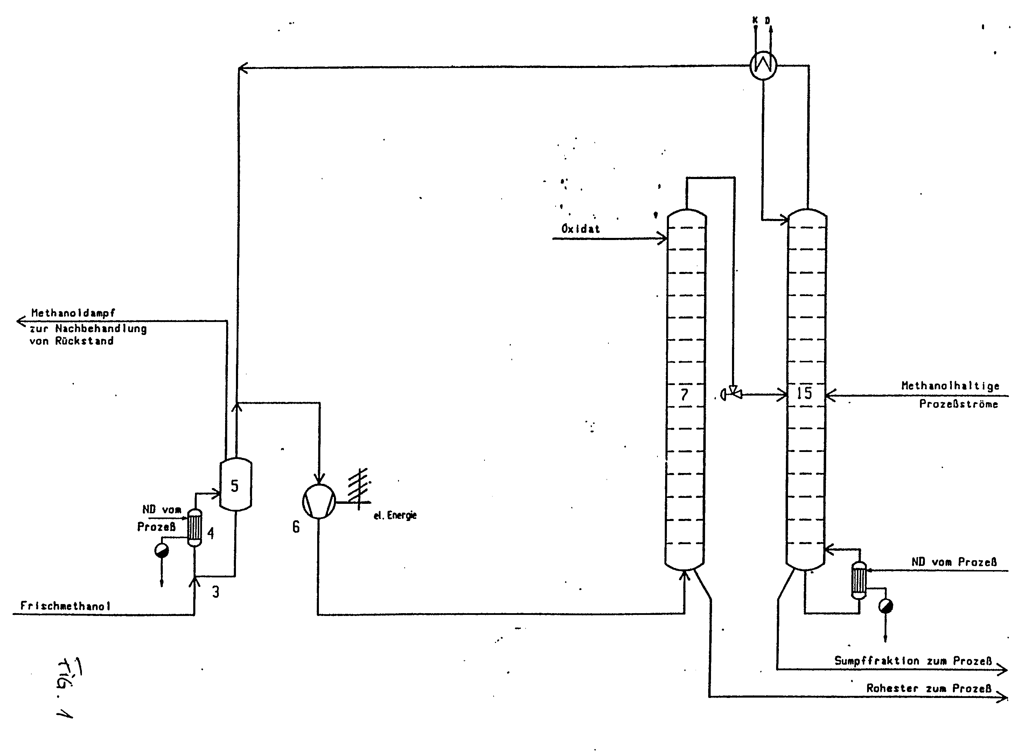

Das erfindungsgemäße Verfahren wird nachfolgend anhand der Figur 1 der Zeichnung weiter erläutert.

Das zum Ersatz der Verluste benötigte Methanol wird in die Leitung 3 als Frisch-Methanol eingespeist. Im Umlaufverdampfer 4 wird das Reaktionsmethanol über den Ausdampfbehälter 5 unter einem Druck von etwa 4 bis 8 bar verdampft. Der Umlaufverdampfer wird mit an anderer Stelle des Verfahrens anfallendem Niederdruckdampf beheizt. Ein Teil des Reaktionsmethanols kann für die reaktive Nachbehandlung von Rückstandsfraktionen aus der destillativen Aufarbeitung des Rohesters einer Rückstandsnachbehandlung zugeführt werden. Das für die Veresterung bestimmte Reaktionsmethanol wird am Kopf des Ausdampfbehälters 5 dampfförmig abgezogen. In einer Rektifikationskolonne 15 werden die Methanol und Reaktionswasser enthaltenden Veresterungsbrüden gemeinsam mit den übrigen methanol-haltigen Prozeßströmen in eine wasserhaltige Sumpffraktion und eine methanol-haltige Kopffraktion zerlegt. Diese Kopffraktion wird zusammen mit dem am Ausdampfbehälter 5 anfallenden Methanoldampf dem Kompressor 6 zugeführt und auf etwa 25 bis 30 bar verdichtet, wobei gleichzeitig eine Überhitzung auf etwa 220 bis 280°C auftritt, und schließlich dem Veresterungsreaktor 7 zugeführt. Das Oxidat wird am Kopf von Reaktor 7 aufgegeben.

Am Sumpf des Veresterungsreaktors 7 wird der Rohester abgezogen, und am Kopf des Reaktors 7 fallen die methanolhaltigen Brüden an.

## Patentansprüche

1. Verfahren zur Herstellung von Terephthalsäure (TPS) über Terephthalsäuredimethylester (DMT) aus p-Xylol (p-X) und Methanol durch Oxidation in flüssiger Phase mit Luftsauerstoff in Gegenwart von gelösten Schwermetallverbindungen als Katalysator eines Gemisches aus p-X und einer überwiegend p-Toluylsäuremethylester (p-TE) enthaltenden in die Oxidation zurückgeführten Fraktion zu einem hauptsächlich p-Toluylsäure (p-TS) und Terephthalsäuremonomethylester (MMT) enthaltenden Oxidat bei einer Temperatur von 140 bis 170° C und einem Druck von 4 bis 8 bar, Veresterung des Oxidats mit auf erhöhten Druck gebrachtem flüssigem und anschließend verdampftem Methanol bei einer Temperatur von 220 bis 280°C und einem Druck von 20 bis 30 bar zu einem hauptsächlich p-TE und DMT enthaltenden Rohester, Abzug der Rohesterfraktion und einer methanolhaltigen Brüdenfraktion aus der Veresterung, destillative Auftrennung des Rohesters in eine p-TE-Fraktion und eine terephthalaldehydsäuremethylesterreiche Fraktion, die in die Oxidation zurückgeführt werden, eine Roh-DMT-Fraktion und eine Rückstands fraktion, Hydrolyse der Roh-DMT-Fraktion mit Wasser, Gewinnung der entstehenden TPS und Abtrennung eines Methanol-Wasser-Gemisches, <u>dadurch gekennzeichnet</u>, daß die methanolhaltige Brüdenfraktion und das Methanol-Wasser-Gemisch durch Rektifikation bei einem Druck von 2 bis 20 bar und bei erhöhter Temperatur in eine methanol-reiche Kopffraktion und eine wäßrige Sumpfphase zerlegt werden, das am Kopf der Rektifikation anfallende Methanol unter Druck dampfförmig entnommen wird und die Veresterung des Oxidats mit dem durch Kompression auf Veresterungsdruck und Veresterungstemperatur gebrachten methanol-haltigen Dampf durchgeführt wird.

2. Verfahren nach Anspruch 1, dadurch gekennzeichnet, daß die aus der Veresterung abgezogenen Brüden gewaschen und anschließend in einer Brüden-Entspannungsturbine zum Antrieb des Hethanolkompressors oder eines Generators auf 0,1 bis 8 bar entspannt und anschließend in das Verfahren zurückgeführt werden.

3. Verfahren nach Anspruch 2, dadurch gekennzeichnet, daß die Brüden vor Eintritt in die Turbine mit Prozeßwasser gewaschen oder teilweise kondensiert werden und ein Teil des Kondensats für die Brüdenwaschung verwendet wird.

## Claims

1. Process for the production of terephthalic acid (TPA) by way of terephthalic acid dimethyl ester (DMT) produced from p-xylene (p-X) and methanol by oxidation with atmospheric oxygen in the presence of dissolved heavy metal compounds as catalyst at a temperature of 140 to 170°C and a pressure of 4 to 8 bar of a mixture of p-X and a fraction containing predominantly p-toluic acid methyl ester (p-TE) led back to the oxidation in the liquid phase to form a chiefly p-toluic acid (p-TA) and terephthalic acid monomethyl ester (MMT)-containing oxidation product, esterification of the oxidation product at a temperature of 220 to 280°C and a pressure of 20 to 30 bar with liquid methanol brought to elevated pressure and then evaporated, to form a chiefly p-TE and DMT-containing crude ester, withdrawal of the crude ester fraction and a methanol-containing vapour fraction from the esterification, distillative separation of the crude ester into a p-TE fraction and a terephthalaldehyde acid methyl ester-rich fraction which are led back to the oxidation, a crude DMT fraction and a residual fraction, hydrolysis of the crude DMT fraction with water, recovery of the TPA which is present and separation off of a methanol-water mixture, characterised in that the methanol-containing vapour fraction and the methanol-water mixture are stripped by rectification at a pressure of 2 to 20 bar and at elevated temperature into a methanol-rich head fraction and an aqueous sump phase, the methanol depositing at the head of the rectification being removed in the form of vapour under pressure and the esterification of the oxidation product being carried out with the methanol-containing vapour brought by compression to esterification pressure and esterification temperature.

2. Process according to claim 1, characterised in that the vapours drawn off from the esterification are washed and then expanded in a vapour expansion turbine for driving of the methanol compressor or a generator at 0.1 to 8 bar and then are led back into the process.

3. Process according to claim 2, characterised in that the vapours are washed with process water before entry into the turbine or are partially condensed and a part of the condensate is used for the washing of the vapours.

**Revendications**

1. Procédé de préparation d'acide téréphtalique (TPS) par l'intermédiaire de téréphtalate de diméthyle (DMT)obtenu à partir de p-xylène (p-X) et de méthanol par oxydation en phase liquide avec de l'oxygène de l'air en présence de composés de métaux lourds dissous comme catalyseur, d'un mélange de p-X et d'une fraction recyclée vers la phase d'oxydation contenant essentiellement du p-toluate de méthyle (p-TE), pour obtenir un produit d'oxydation renfermant principalement de l'acide p-toluique (p-TS) et du téréphthalate monométhylique (MMT) à une tempétature de 140 à 170°C et sous une pression de 4 à 8 bars, par estérification du produit d'oxydation avec du méthanol liquide amené à une pression accrue et aussitôt vaporisé à une température de 220 à 280°C et sous une pression de 20 à 30 bars en donnant un ester brut contenant principalement du p-TE et du DMT, par soutirage à partir de l'etape d'estérification de la fraction d'ester brut et d'une fraction de vapeur contenant du méthanol, par séparation par distillation de l'ester brut en une fraction de p-TE et une fraction riche en aldéhyde téréphtalate de méthyle qui sont recyclées vers la phase d'oxydation, en une fraction de DMT brut et en une fraction résiduelle, par hydrolyse de la fraction de DMT brut avec de l'eau, par récupération du TPS formé et séparation d'un mélange méthanol-eau, caractérisé en ce que la fraction de vapeur méthanolique et le mélange méthanol-eau sont séparés par rectification sous une pression de 2 à 20 bars et à une température accrue en une fraction de tête riche en méthanol et en une phase de queue aqueuse, que le méthanol formé au sommet de la colonne de rectification est soutiré sous pression à l'état de vapeur et que l'estérification du produit d'oxydation s'effectue avec la vapeur méthanolique amenée par compression à la pression et à la température d'estérification.

2. Procédé selon la revendication 1, caractérisé en ce que les vapeurs soutirées de la phase d'estérification sont lavées, consécutivement détendues à 0,1-8 bars dans une turbine de détente des vapeurs en vue de l'entraînement du compresseur de méthanol ou d'un générateur et sont ensuite recyclées dans le procédé.

3. Procédé selon la revendication 2, caractérisé en ce que les vapéurs sont lavées avec de l'éau du procédé ou partiellement condensées avant leur admission dans la turbine et qu'une partie du condensat est utilisée pour le lavage des vapeurs.

Methanolhaltige Prozeßströme

ND vom Prozeß

Sumpffraktion zum Prozeß

Rohester zum Prozeß

15

7

K D

Oxidat

el. Energie

6

5

4

3

ND vom Prozeß

Frischmethanol

Methanoldampf zur Nachbehandlung von Rückstand

FiG. 1